(19) Europäisches Patentamt
European Patent Office
Office européen des brevets

(11) Publication number: **0 175 309**
**A2**

(12) **EUROPEAN PATENT APPLICATION**

(21) Application number: 85111629.3

(22) Date of filing: 13.09.85

(51) Int. Cl.⁴: **C 12 P 13/08**
// (C12P13/08, C12R1:15, 1:13)

(30) Priority: 14.09.84 JP 191710/84
12.10.84 JP 212456/84
06.11.84 JP 233522/84
05.12.84 JP 255592/84
05.12.84 JP 255593/84

(43) Date of publication of application: 26.03.86
Bulletin 86/13

(84) Designated Contracting States: DE FR GB

(71) Applicant: TORAY INDUSTRIES, INC., 2, Nihonbashi Muromachi 2-chome Chuo-ku, Tokyo 103 (JP)

(72) Inventor: Matsumoto, Shun-ich, 5-51, Tsunishi 2-chome, Kamakura-shi, Kanagawa-ken 248 (JP)

(74) Representative: Kador . Klunker . Schmitt-Nilson . Hirsch, Corneliusstrasse 15, D-8000 München 5 (DE)

(54) **A method for producing l-lysine.**

(57) L-lysine can be produced by fermentation at a high yield from Brevibacterium or Corynebacterium mutants which are resistant to at least one of the α-substituted amino-ε-caprolactam compounds.

EP 0 175 309 A2

TORAY INDUSTRIES, INC.
2, Nihonbashi-Muromachi 2-chome
Chuo-ku, Tokyo, 103 Japan



# A METHOD FOR PRODUCING L-LYSINE

## Background of the invention

### (a) Field of the invention

This invention relates to a method for producing L-lysine by fermentation.
L-lysine, which is useful as a feed additive, has been produced by fermentation hitherto.

### (b) Description of the Prior Art

It is disclosed in US Patent Specification No. 3,707,441 and Published Examined Japanese Patent Application No. 45392/1978 that the microbial mutant strains belonging to the genus Brevibacterium or Corynebacterium, of which the mutant is resistant to S-(2-aminoethyl)-L-cystein, or α-chloro-ε-caprolactam, can be used as microorganisms capable of producing L-lysine by fermentation.
These prior methods are superior for industrial use. However, there is room for further improvement in the capability of the strain as to the yield of L-lysine.

## Summary of the invention

One object of the present invention is to provide an improved method for producing L-lysine by fermentation which can give a much higher yield.
This and other objects of the invention will become more apparent in the detailed description and examples hereinafter.

These objects are attained by a method for producing L-lysine which comprises the steps of:

(a) culturing an L-lysine producing microorganism belonging to the genus Corynebacterium or Brevebacterium until L-lysine is accumulated in a culture medium, said microorganism being resistant to at least one of the α-substituted amino-ε-caprolactam compounds shown by the following formula (I);

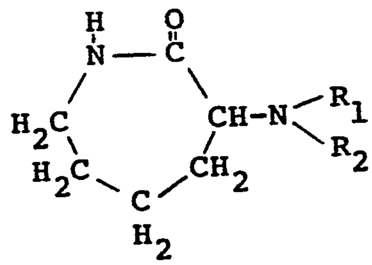

(I)

wherein $R_1$ and $R_2$ are the same or different and are a hydrogen atom or an organic radical having from one to four carbon atoms, and

(b) recovering the accumulated L-lysine from the culture medium.

Description of the preferred embodiments

The microorganisms used in the present invention belong to the genus Brevibacterium or Corynebacterium and these microorganisms are resistant to at least one of the α-substituted amino-ε-caprolactam compounds.

The α-substituted amino-ε-caprolactam compounds used in the invention are the compounds shown by the following formula (I);

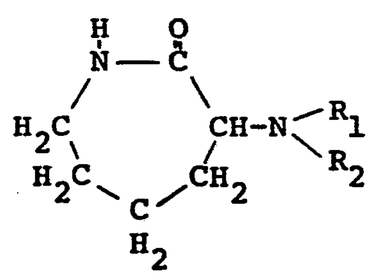

(I)

0 175 309

wherein $R_1$ and $R_2$ are the same or different and are a hydrogen atom or an organic radical having from one to four carbon atoms.

$R_1$ and $R_2$ in the above formula (I) are preferably each selected from the group consisting of a hydrogen atom, an alkyl radical, a 2-hydroxyethyl radical, a 3-chloro-2-hydroxypropyl radical and a 2,3-epoxypropyl radical.

Most suitable $\alpha$-substituted amino-$\varepsilon$-caprolactams of the invention are shown as follows;

(1)

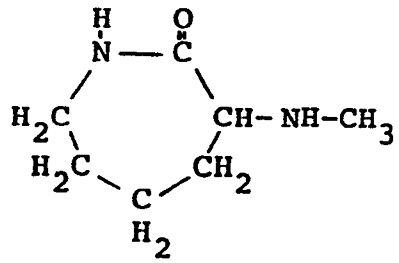

(2)

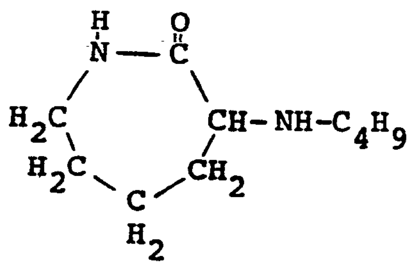

(3)

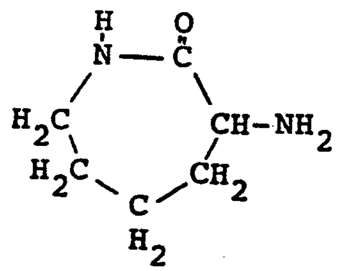

(4)

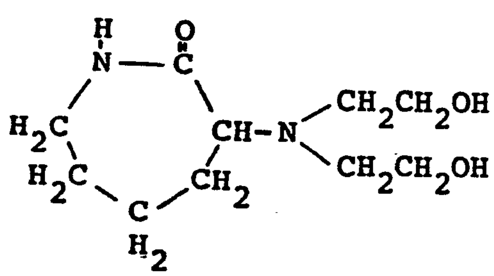

(5)

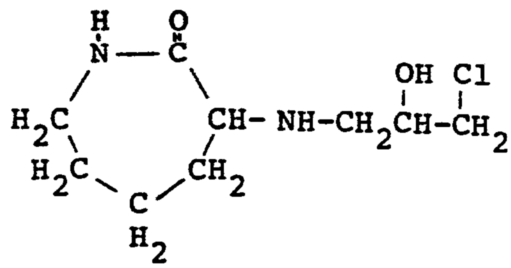

(6)

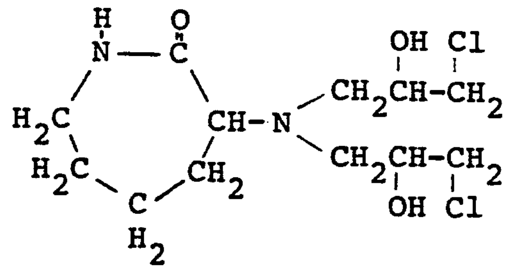

(7)

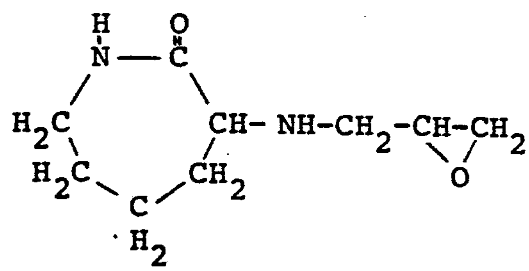

The specimens of the microorganisms used in the invention are as follows;

(a) Corynebacterium glutamicum TM-1103(FERM BP-882) (resistant to

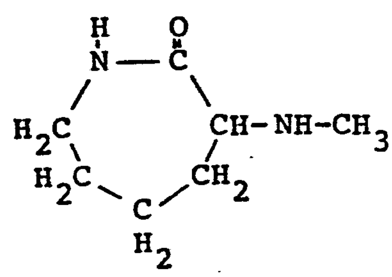

)

(b) Corynebacterium glutamicum TM-1120(FERM P-7701) (resistant to

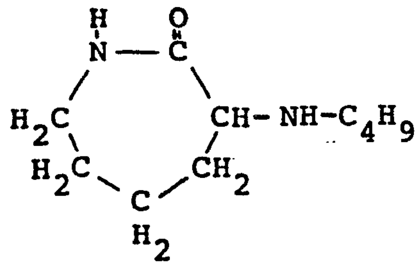

)

(c) Corynebacterium glutamicum TM-1107(FERM BP-885) (resistant to

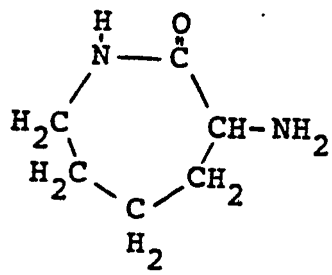

)

(d) Corynebacterium glutamicum TM-1104(FERM BP-883) (resistant to

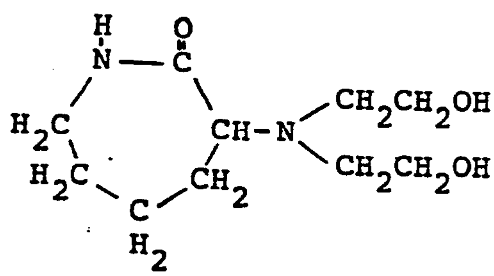

)

(e) Corynebacterium glutamicum TM-1105(FERM P-7691) (resistant to

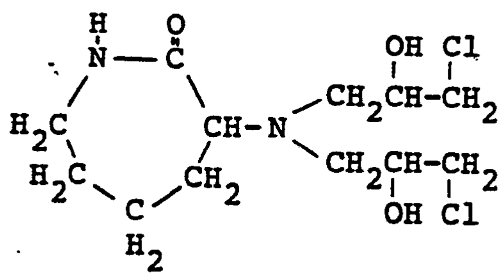

)

0 175 309

(f) Corynebacterium glutamicum TM-1101 (FERM BP-880) resistant to 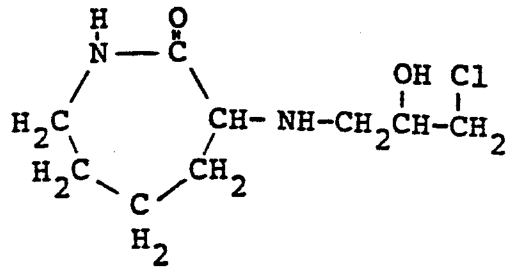 )

(g) Corynebacterium glutamicum TM-1115(FERM BP-886) (resistant to 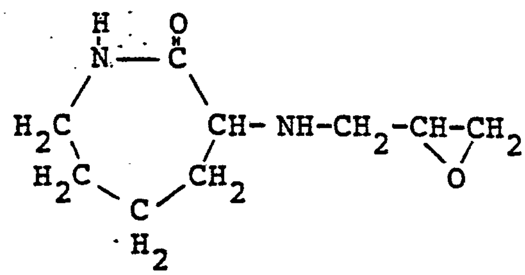 )

(h) Brevibacterium lactofermentum TM-1113(FERM P-7696) (resistant to 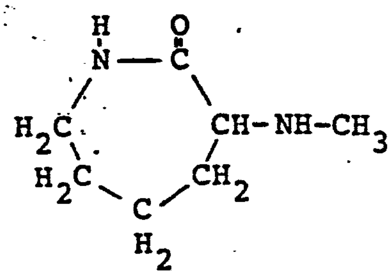 )

(i) Brevibacterium lactofermentum TM-1121(FERM P-7702) (resistant to 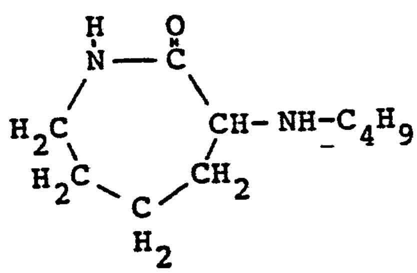 )

(j) Brevibacterium lactofermentum TM-1108(FERM P-7694) (resistant to 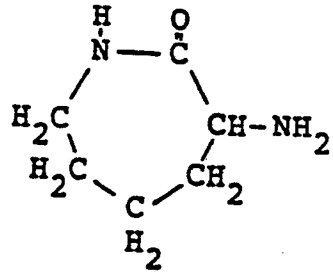 )

(k) Brevibacterium lactofermentum TM-1106(FERM BP- 884 ) (resistant to 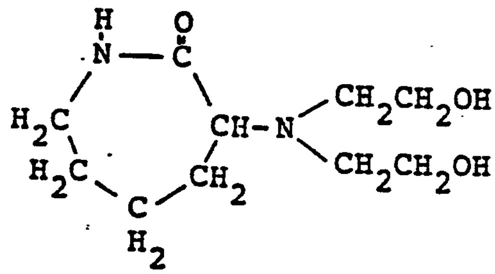 )

(l) Brevibacterium lactofermentum TM-1114 (FERM P-7697) (resistant to 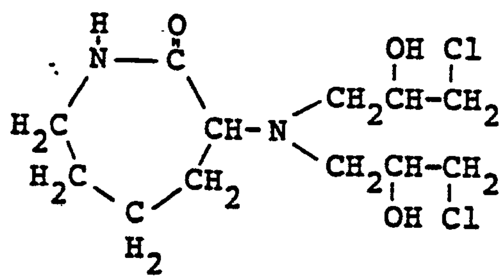 )

(m) Brevibacterium lactofermentum TM-1102(FERM BP- 881) (resistant to 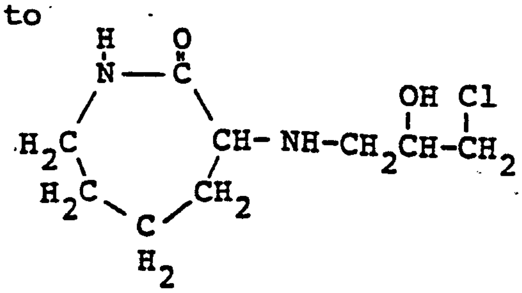 )

(n) Brevibacterium lactofermentum TM-1116(FERM P-7699) (resistant to )

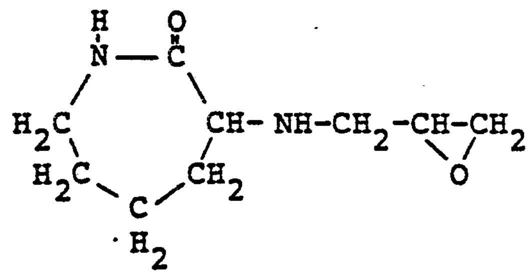

The FERM-P numbers and FERM-BP numbers are the access numbers of the Fermentation Research Institute Agency of Industrial Science and Technology, at No. 1-3, Yatabe-cho Higashi 1-chome, Tsukuba-gun, Ibaragi-ken, 305 Japan, from which the microorganisms with the FERM-P and FERM-BP numbers are freely available to any party who requests them.

These lysine producing microorganisms can be induced as a mutant, for example, from the following parent strains of the genera Corynebacterium and Brevibacterium;

| | |
|---|---|
| Corynebacterium glutamicum (Micrococcus glutamicus) | ATCC 13286 |
| Brevibacterium lactofermentum | ATCC 13 869 |

Methods for inducing and separating the mutants of the invention are conventional methods such as treatment with ultraviolet light, X-rays, $\gamma$-rays, and N-methyl-N'-nitro-N-nitrosoguanidine.

The following experiments show by way of example the methods for inducing and separating the mutants.

Experiment 1

Corynebacterium glutamicum ATCC 13286 (hereinafter referred to as TM-1117) is exposed to ultraviolet light by a conventional method. The exposed strains

are spread on the agar plate medium, which is the minimum medium, to which 0.2% of a α-monomethylamino-ε-caprolactam (hereinafter referred to as MAC) is added and they are cultured for 4 to 10 days at 30°C. The minimum medium is shown in Table 1.

Table 1

| | |
|---|---|
| Minimum medium | |
| Glucose | 10 g/ℓ |
| $(NH_4)_2SO_4$ | 1.5 g/ℓ |
| Urea | 1.5 g/ℓ |
| $KH_2PO_4$ | 1.0 g/ℓ |
| $K_2HPO_4$ | 3.0 g/ℓ |
| $MgSO_4 \cdot 7H_2O$ | 0.1 g/ℓ |
| $CaCl_2 \cdot 2H_2O$ | 0.001 g/ℓ |
| NaCl | 0.005 g/ℓ |
| $FeSO_4 \cdot 7H_2O$ | 0.005 g/ℓ |
| $MnSO_4 \cdot 4H_2O$ | 0.005 g/ℓ |
| Biotin | $30 \times 10^{-6}$ g/ℓ |
| Thiamine · HCl | $100 \times 10^{-6}$ g/ℓ |
| Polypeptone S | 0.003 g/ℓ |
| Agar | 20 g/ℓ |
| (pH = 6.8) | |

Colonies formed on the plate medium are separated, and the most effective lysine producing mutant TM-1103 is separated from the colonies.

Experiment 2

In a similar manner to Experiment 1, except that L-α-amino-ε-caprolactam (hereinafter referred to as LAC) is added, the most effective lysine producing mutant TM-1107 is separated.

Experiment 3

The strain TM-1117 is exposed to NTG by a conventional method. The exposed strains are spread and cultured in a similar manner to Experiment 1 except that α-monobutylamino-ε-caprolactam (hereinafter referred to as BAC) is added instead of MAC. The most effective lysine producing mutant TM-1120 is separated from the colonies.
In a similar manner to the above mentioned method except that α-bis(2-hydroxyethyl)amino-ε-caprolactam (herinafter referred to as BHC), α-(3-chloro-2-hydroxypropyl)amino-ε-caprolactam (hereinafter referred to as MCC), α-bis(3-chloro-2-hydroxypropyl)amino-ε-caprolactam (hereinafter referred to as BCC), and α-(2,3-epoxypropyl)amino-ε-caprolactam (hereinafter referred to as EPC) are added in each case instead of BAC, the most effective lysine producing mutants TM-1104, TM-1101, TM-1105, and TM-1115, are separated, respectively.

Experiment 4

Brevibacterium lactofermentum ATCC 13869 (hereinafter referred to as TM-1118) is exposed to N-methyl-N'-nitro-N-nitrosoguanidine (hereinafter referred to as NTG) by a conventional method.
The exposed strains are spread and cultured in the same manner as in Experiment 1. From the colonies, a lysine producing mutant which requires homoserine is separated. Thereafter, the resulting mutant is exposed to NTG, and spread and cultured again in the same manner as in Experiment 1.
From the colonies, the most effective lysine producing mutant TM-1113 is separated.
In a similar manner to the above mentioned method except that LAC, BCC, MCC, BHC, and EPC are added in each case instead of MAC, the most effective lysine producing mutants TM-1108, TM-1114, TM-1102, TM-1106, and TM-1116 are separated, respectively.

Experiment 5

The strain TM-1118 is exposed to NTG by a conventional method. The exposed strains are spread and cultured in a similar manner to Experiment 1 except that BAC is added instead of MAC. From the colonies, the most effective lysine producing mutant TM-1121 is separated.

Experiment 6

The medium is prepared by adding each α-amino-ε-caprolactam derivative to the culture medium shown in Table 2. Each of the microorganisms shown in Table 3 is inoculated into 10 ml of the above mentioned medium and cultured aerobically in a test tube at 30°C for 48 hours.

After cultivation, the growth degree is determined by measuring the optical density at 550 mμ of the culture broth diluted to 2 times. The results are shown in Table 3.

Table 2

| Culture medium | |
|---|---|
| Glucose | 15 g/ℓ |
| $(NH_4)_2SO_4$ | 2.0 g/ℓ |
| Urea | 2.0 g/ℓ |
| $KH_2PO_4$ | 2.0 g/ℓ |
| $K_2HPO_4$ | 1.0 g/ℓ |
| $MgSO_4 \cdot 7H_2O$ | 0.1 g/ℓ |
| $CaCl_2 \cdot 2H_2O$ | 0.001 g/ℓ |
| NaCl | 0.005 g/ℓ |
| $FeSO_4 \cdot 7H_2O$ | 0.005 g/ℓ |
| $MnSO_4 \cdot 4H_2O$ | 0.005 g/ℓ |
| d-Biotin | $50 \times 10^{-6}$ g/ℓ |
| Thiamine·HCl | $100 \times 10^{-6}$ g/ℓ |
| Polypeptone S | 0.003 g/ℓ |
| (pH = 6.8) * | |

* The pH of the culture medium is adjusted to 6.8 by NaOH.

Table 3

| Microorganisms | α-Amino-ε-caprolactam derivatives | Added amount (g/ℓ) | Growth degree |
|---|---|---|---|
| TM-1117 | None | 0 | 0.413 |
| TM-1117 | MAC | 1.0 | 0.091 |
| TM-1117 | MAC | 2.0 | 0.045 |
| TM-1103 | None | 0 | 0.425 |
| TM-1103 | MAC | 1.0 | 0.275 |
| TM-1103 | MAC | 2.0 | 0.155 |
| TM-1117 | BAC | 1.0 | 0.165 |
| TM-1117 | BAC | 2.0 | 0.092 |
| TM-1120 | None | 0 | 0.405 |
| TM-1120 | BAC | 1.0 | 0.223 |
| TM-1120 | BAC | 2.0 | 0.159 |
| TM-1117 | LAC | 1.0 | 0.081 |
| TM-1117 | LAC | 2.0 | 0.052 |
| TM-1107 | None | 0 | 0.451 |
| TM-1107 | LAC | 1.0 | 0.215 |
| TM-1107 | LAC | 2.0 | 0.102 |
| TM-1117 | BHC | 1.0 | 0.112 |
| TM-1117 | BHC | 2.0 | 0.090 |

1

Table 3 (Continued)

| Microorganisms | α-Amino-ε-caprolactam derivatives | Added amount (g/ℓ) | Growth degree |
|---|---|---|---|
| TM-1104 | None | 0 | 0.481 |
| TM-1104 | BHC | 1.0 | 0.285 |
| TM-1104 | BHC | 2.0 | 0.210 |
| TM-1117 | MCC | 1.0 | 0.101 |
| TM-1117 | MCC | 2.0 | 0.082 |
| TM-1101 | None | 0 | 0.492 |
| TM-1101 | MCC | 1.0 | 0.255 |
| TM-1101 | MCC | 2.0 | 0.155 |
| TM-1117 | BCC | 1.0 | 0.113 |
| TM-1117 | BCC | 2.0 | 0.092 |
| TM-1105 | None | 0 | 0.435 |
| TM-1105 | BCC | 1.0 | 0.215 |
| TM-1105 | BCC | 2.0 | 0.162 |
| TM-1117 | EPC | 1.0 | 0.101 |
| TM-1117 | EPC | 2.0 | 0.082 |
| TM-1115 | None | 0 | 0.459 |
| TM-1115 | EPC | 1.0 | 0.212 |
| TM-1115 | EPC | 2.0 | 0.185 |

Table 3 (Continued)

| Microorganisms | α-Amino-ε-caprolactam derivatives | Added amount (g/ℓ) | Growth degree |
|---|---|---|---|
| TM-1118 | None | 0 | 0.389 |
| TM-1118 | MAC | 1.0 | 0.081 |
| TM-1118 | MAC | 2.0 | 0.052 |
| TM-1113 | None | 0 | 0.395 |
| TM-1113 | MAC | 1.0 | 01266 |
| TM-1113 | MAC | 2.0 | 0.118 |
| TM-1118 | BAC | 1.0 | 0.118 |
| TM-1118 | BAC | 2.0 | 0.080 |
| TM-1121 | None | 0 | 0.376 |
| TM-1121 | BAC | 1.0 | 0.266 |
| TM-1121 | BAC | 2.0 | 0.172 |
| TM-1118 | LAC | 1.0 | 0.097 |
| TM-1118 | LAC | 2.0 | 0.082 |
| TM-1108 | None | 0 | 0.388 |
| TM-1108 | LAC | 1.0 | 0.181 |
| TM-1108 | LAC | 1.0 | 0.122 |
| TM-1118 | BHC | 1.0 | 0.097 |
| TM-1118 | BHC | 2.0 | 0.081 |

Table 3 (Continued)

| Microorganisms | α-Amino-ε-caprolactam derivatives | Added amount (g/ℓ) | Growth degree |
|---|---|---|---|
| TM-1106 | None | 0 | 0.522 |
| TM-1106 | BHC | 1.0 | 0.269 |
| TM-1106 | BHC | 2.0 | 0.176 |
| TM-1118 | MCC | 1.0 | 0.099 |
| TM-1118 | MCC | 2.0 | 0.093 |
| TM-1102 | None | 0 | 0.512 |
| TM-1102 | MCC | 1.0 | 0.225 |
| TM-1102 | MCC | 2.0 | 0.182 |
| TM-1118 | BCC | 1.0 | 0.102 |
| TM-1118 | BCC | 2.0 | 0.100 |
| TM-1114 | None | 0 | 0.532 |
| TM-1114 | BCC | 1.0 | 0.241 |
| TM-1114 | BCC | 2.0 | 0.199 |
| TM-1118 | EPC | 1.0 | 0.112 |
| TM-1118 | EPC | 2.0 | 0.091 |
| TM-1116 | None | 0 | 0.422 |
| TM-1116 | EPC | 1.0 | 0.192 |
| TM-1116 | EPC | 2.0 | 0.138 |

The methods for producing L-lysine using the microorganisms mentioned above are conventional, and the microorganisms are cultured in a conventional medium containing carbon sources, nitrogen sources, inorganic salts, nutrients required for growth, and other minor nutrients.

As the carbon source, carbohydrates such as glucose, sucrose, molasses, starch hydrolysate, or cellulose hydrolysate derived from the dregs of juice or fruit, pulp or paper, organic acid such as acetic acid, alcohol such as ethanol, hydrocarbons, etc., can be used. In the invention molasses can be preferably used.

As the nitrogen source, ammonia gas, organic ammonium compound such as ammonium acetate, inorganic ammonium compound such as ammonium sulfate or ammonium chloride, urea, etc., can be used.

As the inorganic salt, potassium dihydrogen phosphate, potassium monohydrogen phosphate, magnesium sulfate, ferrous sulfate, calcium carbonate, etc., can be used.

As a nutrient, natural organic nutrients such as vitamins, amino acids, corn steep liquor, meat extract, yeast extract, peptone or soy bean hydrolysate can be preferably used.

Cultivation is carried out under aerobic conditions at a temperature of from 24°C to 40°C for 2 to 7 days. During cultivation the pH of the medium is adjusted to 5.5 to 8.5 by alkali or acid or calcium carbonate or gaseous ammonia.

The carbon source is in general preferably added by the so-called feeding method. In this case, L-lysine is usually accumulated in the medium after 2 to 5 days.

L-lysine in the culture broth thus obtained can be separated by known methods such as by using ion-exchange resins or other conventional methods, after removal of the microbial cells by filtration or centrifuging.

Depending on the kind of medium, L-lysine can be directly obtained in the form of crystals from the medium.

The invention will be more clearly understood with reference to the following Examples. However, these examples are intended to illustrate the invention and are not to be construed as limiting the scope of the invention.

Example 1

150 ml of a seed culture medium shown in Table 4 is placed in a 1 ℓ Erlenmeyer flask, and inoculated with each of the microorganisms shown in Table 6. Each microorganism is cultivated at a temperature of 30°C for 24 hours, being shaken.

Thus the seed culture broth is obtained.

**Table 4**

| Seed culture medium | |
|---|---|
| Meat extract | 5 g/ℓ |
| Peptone | 15 g/ℓ |
| NaCl | 5 g/ℓ |
| $KH_2PO_4$ | 5 g/ℓ |

850 ml of a main culture medium shown in Table 5 and 150 ml of the above mentioned seed culture broth are placed in 2 ℓ of fermentor.

Cultivation is carried out at 30°C for 70 to 90 hours under aerobic conditions.

The accumulated amount of L-lysine is shown in Table 6.

Table 5

| Main culture medium | |
| --- | --- |
| Glucose | 110 g/ℓ |
| $(NH_4)_2SO_4$ | 50 g/ℓ |
| $KH_2PO_4$ | 1.2 g/ℓ |
| $MgSO_4 \cdot 7H_2O$ | 0.5 g/ℓ |
| $FeSO_4 \cdot 7H_2O$ | 0.005 g/ℓ |
| $MnCl_2 \cdot 4H_2O$ | 0.005 g/ℓ |
| $CaCl_2 \cdot 2H_2O$ | 0.005 g/ℓ |
| NaCl | 0.005 g/ℓ |
| d-Biotin | $50 \times 10^{-6}$ g/ℓ |
| Thiamine·HCl | $200 \times 10^{-6}$ g/ℓ |
| Polypeptone S | 15 g/ℓ |
| Antibubbling agent | 0.19 g/ℓ |

0 175 309

Table 6

| Microorganism | L-lysine accumulated (g/ℓ)* |
|---|---|
| TM-1117 | 32.7 |
| TM-1103 | 44.3 |
| TM-1120 | 42.7 |
| TM-1107 | 46.1 |
| TM-1104 | 39.2 |
| TM-1101 | 44.8 |
| TM-1105 | 46.2 |
| TM-1115 | 39.2 |
| TM-1118 | 0 |
| TM-1113 | 39.1 |
| TM-1121 | 36.5 |
| TM-1108 | 39.2 |
| TM-1106 | 39.8 |
| TM-1102 | 39.8 |
| TM-1114 | 41.8 |
| TM-1116 | 38.5 |

* The amount is calculated from L-lysine · HCl.

Example 2

Cultivation is carried out in a similar manner to Example 1 except that the main culture medium shown in Table 7 is used instead of the main culture medium shown in Table 5. Cultivation is carried out for 90 to 110 hours. The accumulated amount of L-lysine is shown in Table 8.

Table 7

| Main culture medium | |
|---|---|
| molasses* | 220 g/ℓ |
| $(NH_4)_2SO_4$ | 50 g/ℓ |
| Polypeptone S | 15 g/ℓ |

* The amount calculated as sugar is 110 g/ℓ . The molasses is pretreated by being heated with a little $H_2SO_4$ at 120°C for 10 minutes.

Table 8

| Microorganism | L-lysine accumulated (g/ℓ)* |
|---|---|
| TM-1117 | 38.3 |
| TM-1107 | 48.2 |
| TM-1104 | 42.5 |
| TM-1101 | 49.2 |
| TM-1105 | 45.6 |
| TM-1115 | 42.1 |
| TM-1118 | 0 |
| TM-1113 | 39.1 |
| TM-1121 | 39.9 |
| TM-1108 | 42.1 |
| TM-1106 | 42.8 |
| TM-1114 | 43.8 |
| TM-1116 | 40.8 |

* The amount is calculated from L-lysine · HCl.

What we claim is;

1. A method for producing L-lysine which comprises the steps of:

(a) culturing an L-lysine producing microorganism belonging to the genus Corynebacterium or Brevebacterium until L-lysine is accumulated in a culture medium, said microorganism being resistant to at least one of the α-substituted amino-ε-caprolactam compounds shown by the following formula (I);

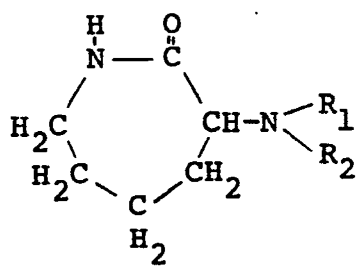

(I)

wherein $R_1$ and $R_2$ are the same or different and are a hydrogen atom or an organic radical having from one to four carbon atoms, and

(b) recovering the accumulated L-lysine from the culture medium.

2. A method according to claim 1, wherein said $R_1$ and $R_2$ are each selected from the group consisting of a hydrogen atom, an alkyl radical, a 2-hydroxyethyl radical, a 3-chloro-2-hydroxypropyl radical and a 2,3-epoxypropyl radical.